(19) Europäisches Patentamt European Patent Office Office européen des brevets

(11) EP 3 047 799 A1

(12) EUROPEAN PATENT APPLICATION
published in accordance with Art. 153(4) EPC

(43) Date of publication:
27.07.2016 Bulletin 2016/30

(51) Int Cl.:
A61B 6/03 (2006.01)  G01N 23/083 (2006.01)
A61B 6/00 (2006.01)  A61B 6/02 (2006.01)

(21) Application number: 14879003.3

(22) Date of filing: 17.01.2014

(86) International application number:
PCT/CN2014/000057

(87) International publication number:
WO 2015/106365 (23.07.2015 Gazette 2015/29)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME

(71) Applicant: Beijing East Whale Image Technology
Co., Ltd.
Haidian District, Beijing 100176 (CN)

(72) Inventors:
• ZHU, Xun
Beijing 100176 (CN)

• HE, Xingbai
Beijing 100176 (CN)
• ZHANG, Jun
Beijing 100176 (CN)

(74) Representative: Hanna, Peter William Derek et al
Hanna Moore + Curley
Garryard House
25/26 Earlsfort Terrace
Dublin 2, D02 PX51 (IE)

(54) X-RAY PARADOXICAL PULSE BI-PLANAR SYNCHRONOUS REAL-TIME IMAGING DEVICE AND IMAGING METHOD

(57) An X-ray paradoxical pulse bi-planar synchronous real-time imaging device and imaging method are disclosed in this invention, wherein the device comprises the second C-arm and the first C-arm slidably set on the said second C- arm, wherein the first X-ray generator and the first image receiver are mounted on the said first C-arm, and the second X-ray generator and the second image receiver are mounted on the said second C-arm. An imaging control device controls the pulse cycle and width of the said first X-ray pulse and second X-ray pulse, so that the said first X-ray pulse and second X-ray pulse do not mutually interfere or have low degree of interference in one cycle. Therefore, the synchronous real-time X-ray imaging of the said first imaging device and second imaging device can be realized. Furthermore, the said imaging control device can display the X-ray images in real time and synchronously by processing the said X-ray images.

Figure 1

## Description

## Technical field

The invention relates to an X-ray imaging device and imaging method, especially relating to an X-ray paradoxical pulse bi-planar synchronous real-time imaging device and imaging method.

## Background

When conducting the operations such as bone fixation operation, the surgeons often need to observe the precise location of metallic implants or mobile surgical instruments for more precise positioning.

The United States Patent US4884293 describes a bi-planar imaging method, which is adopted with two sets of completely independent imaging systems or two C-arms. One set of the imaging system is fixed on the ground of the operating room and another set of system is suspended from the guide rail set at the ceiling. Although the two sets of imaging systems are installed cooperated with each other, the weakness of this method is that the imaging exposure of the two sets of imaging systems is respectively independent and can't be synchronous.

European Patent EP0917856A1 describes another bi-planar imaging method, by which two sets of imaging systems image on two displays one by one after sequential exposure. The method solves the problems of interference and scattering of two-way X-rays, but it can't display the real-time images of two angles synchronously.

## Summary of the invention

The first purpose of the invention is to provide an X-ray paradoxical pulse bi-planar synchronous real-time imaging device that can display the X-ray image in real time and synchronously.

The second purpose of the invention is to provide an X-ray paradoxical pulse bi-planar synchronous real-time imaging method that can display the X-ray image in real time and synchronously.

The technical proposal adopted in the invention to solve the first technical problem is as follows: a kind of X-ray paradoxical pulse bi-planar synchronous real-time imaging device, including the first C-arm, the second C-arm, support device, the first X-ray generator, the first image receiver, the second X-ray generator, the second image receiver and imaging control device;

The said first C-arm is slidably set on the said second C-arm; the said second C-arm is slidably set on the said support device;

The said first X-ray generator and the first image receiver are fixed on two axial ends of the said first C-arm respectively;

The said second X-ray generator and the second image receiver are fixed on two axial ends of the said second C-arm respectively;

Taking the intersecting line between the x-ray emission plane of the said first X-ray generator and the x-ray emission plane of the said second X-ray generator as the rotation axis, the said first X-ray generator and the first image receiver rotate along the rotation axis driven by the said first C-arm and the said second X-ray generator and the second image receiver also rotate along the rotation axis driven by the said second C-arm;

The said imaging control device includes: console, image processor, X-ray controller, the first high-voltage generator, the second high-voltage generator, network card, the first camera, the second camera, the first display, the second display and operation display;

The said image processor and X-ray controller are connected to the signal of the said console; The said first high-voltage generator and second high-voltage generator are connected to the signal of the said X-ray controller and also connected to the first X-ray generator and the second X-ray generator respectively;

The said first camera and second camera are respectively connected to the said image processor and X-ray controller by the signal of the said network card;

The said first display, second display and operation display are connected to the signal of the said image processor respectively.

Alternatively, the said first X-ray generator is the first X-ray bulb tube and the said second X-ray generator is the second X-ray bulb tube.

Alternatively, the said X-ray paradoxical pulse bi-planar synchronous real-time imaging device also includes the first motor set on the said second C-arm, which is connected to the said console by the motor control signal.

Alternatively, the said X-ray paradoxical pulse bi-planar synchronous real-time imaging device also includes a foot switch, which is connected to the said console by signal.

The technical proposal adopted in the invention to solve the second technical problem is as follows: a method to realize the X-ray paradoxical pulse bi-planar synchronous real-time imaging by using the said X-ray paradoxical pulse bi-planar synchronous real-time imaging device, including the following steps:

S10. Control the motion of the said first motor to make the included angle between the directions of the X-ray generated from the first X-ray generator and the X-ray generated from the second X-ray generator a preset value;

S20. Select the operating mode of the said X-ray paradoxical pulse bi-planar synchronous real-time imaging device;

S30. The said first high-voltage generator and the second high-voltage generator are controlled by the said X-ray controller to generate the pulse high voltage. The said pulse high voltages generated by the said first high-voltage generator and the second high-voltage generator make the said first X-ray generator and second X-ray generator produce X-ray pulses, among which the X-ray pulse produced by the said first X-ray generator is the first X-ray pulse and the X-ray pulse produced by the said second X-ray generator is the second X-ray pulse. The phase of the said first X-ray pulse and that of the second X-ray pulse are different;

S40. The said first image receiver and second image receiver respectively receive the X-ray pulses generated when the first X-ray pulse and second X-ray pulse penetrate the detection object and generate the visible light signals;

S50. The said first camera and second camera respectively collect the visible light signals generated by the first image receiver and second image receiver and transfer to the said image processor by the network card;

S60. The said image processor displays the visible light signals collected by the said first camera on the first display and visible light signals collected by the said second camera on the second display.

Alternatively, in the said S20, the said operating modes include:

a. The first X-ray generator and the first image receiver operate, while the second X-ray generator and the second image receiver do not operate;

b. The second X-ray generator and the second image receiver operate, while the first X-ray generator and the first image receiver do not operate;

c. The first X-ray generator and the first image receiver as well as the second X-ray generator and the second image receiver operate simultaneously.

Alternatively, there is also S05 before the S10, S05. Set the cycle top limit T of the said X-ray paradoxical pulse bi-planar synchronous real-time imaging device.

Alternatively, there is also S35 between the S30 and S40: the said first X-ray pulse's total cycle and the cycle top limit are the same, with the duration as $T_1$; the said second X-ray pulse's total cycle and the cycle top limit are the same, with the duration as $T_2$. The overlap between the said first X-ray pulse and the said second X-ray is Tt, satisfying $T_1 + T_2 \leq T + Tt$.

Alternatively, there is also S36 between the S30 and S40: the said first X-ray pulse's total cycle and the cycle top limit are the same, with the duration as $T_1$; the said second X-ray pulse's total cycle and the cycle top limit are the same, with the duration as $T_2$, satisfying $T_1 + T_2 \leq T$.

Alternatively, in the said S35 or S36, the $T_1$ and $T_2$ are different.

The X-ray paradoxical pulse bi-planar synchronous real-time imaging device of the invention has the following beneficial effects: the said X-ray paradoxical pulse bi-planar synchronous real-time imaging device includes the second C-arm and the first C-arm slidably set on the said second C-arm. The first X-ray generator and the first image receiver are installed on both sides of the said first C-arm, constituting the first imaging device. The second X-ray generator and the second image receiver are installed on both sides of the said second C-arm, constituting the second imaging device. The said imaging control device can control the pulse cycle and width of the said first X-ray pulse and second X-ray pulse, so that the said first X-ray pulse and second X-ray pulse do not mutually interfere or have low interference degree in one cycle. Therefore, the synchronous real-time X-ray imaging of the said first imaging device and second imaging device can be realized. Further, the said imaging control device can display the X-ray images in real time and synchronously by processing the said X-ray images.

The X-ray paradoxical pulse bi-planar synchronous real-time imaging method of the invention has the following beneficial effects: the said first X-ray generator and second X-ray generator are controlled by the said X-ray controller to generate the first X-ray pulse and second X-ray pulse, among which the phases of the said first X-ray pulse and second X-ray pulse are different. Therefore, the said first X-ray pulse and second X-ray pulse do not mutually interfere or have low the interference degree in one cycle. Hence the synchronous real-time X-ray imaging of the said first imaging device and second imaging device can be realized. Further, the said imaging control device can display the X-ray images in real time and synchronously by processing the said X-ray images.

**Brief description of the drawings**

Figure 1 is the structure diagram of the X-ray paradoxical pulse bi-planar synchronous real-time imaging device of the invention;

Figure 2 is the structure diagram of the imaging control device of the invention;

Figure 3 is a sequence diagram of the first X-ray pulse and second X-ray pulse of the invention;

Figure 4 is another sequence diagram of the first X-ray pulse and second X-ray pulse of the invention;

The marks in the figures are as follows: 1 - the first C-arm; 2 - the second C-arm; 3 - the first X-ray generator; 4 - the first image receiver; 5 - the second X-ray generator; 6 - the second image receiver; 7 - imaging control device; 71 - console; 72 - image processor; 73 - X-ray controller; 74 - the first high-voltage generator; 75 - the second high-voltage generator; 76 - network card; 77 - the first camera; 78 - the second camera; 79 - the first display; 710 - the second display; 711 - operation display; 712 - motor controller; 713 - foot switch; 8 - the first motor.

**Detailed description of preferred embodiments**

The technical proposals of the invention are further expounded with the combination of embodiments and attached figures.

Embodiment 1

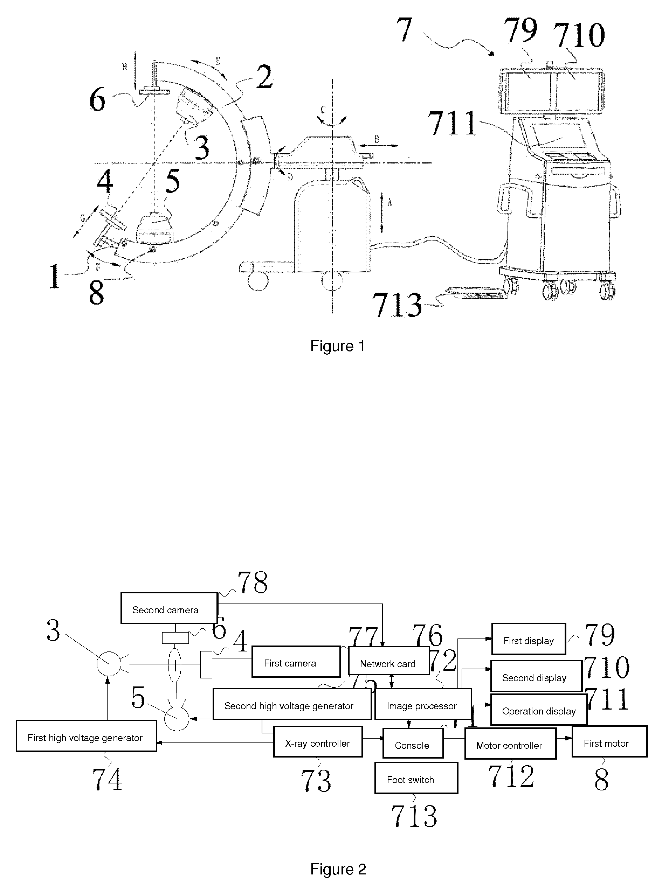

By reference to Figure 1-4, this embodiment provides an X-ray paradoxical pulse bi-planar synchronous real-time imaging device, including the first C-arm 1, the second C-arm 2, support device, the first X-ray generator 3, the first image receiver 4, the second X-ray generator 5, the second image receiver 6 and imaging control device 7;
The said first C-arm 1 is slidably set on the said second C-arm 2 and the said second C-arm 2 is slidably set on said support device;
The said first X-ray generator 3 and the first image receiver 4 are respectively fixed on the two circumferential ends of the first C-arm 1;
The said second X-ray generator 5 and the second image receiver 6 are respectively fixed on the two circumferential ends of the first C-arm 2;
Taking the intersecting line between the x-ray emission plane of the said first X-ray generator 3 and the x-ray emission plane of the said second X-ray generator 5 as the rotation axis, the said first X-ray generator 3 and the first image receiver 4 rotate along the rotation axis driven by the said first C-arm 1 and the said second X-ray generator 5 and the second image receiver 6 also rotate along the rotation axis driven by the said second C-arm 2;
The said imaging control device 7 includes: console 71, image processor 72, X-ray controller 73, the first high-voltage generator 74, the second high-voltage generator 75, network card 76, the first camera 77, the second camera 78, the first display 79, the second display 710 and operation display 711;
The said image processor 72 and X-ray controller 73 are connected to the signal of the said console 71;
The said first high-voltage generator 74 and second high-voltage generator 75 are connected to the signal of the said X-ray controller 73 and connected to the first X-ray generator 3 and the second X-ray generator 5 respectively;
The said first camera 77 and second camera 78 are respectively connected to the said image processor 72 and X-ray controller 73 by the signal of the said network card 76;
The said first display 79, second display 710 and operation display 711 are connected to the signal of the said image processor 72 respectively.
The technical proposal adopted in the invention to solve the second technical problem is as follows: the said X-ray paradoxical pulse bi-planar synchronous real-time imaging device includes the second C-arm 2 and the first C-arm 1 slidably set on the said second C-arm 2. The first X-ray generator 3 and the first image receiver 4 are installed on both sides of the said first C-arm 1, constituting the first imaging device. The second X-ray generator 5 and the second image receiver 6 are installed on both sides of the said second C-arm 2, constituting the second imaging device. The said imaging control device can control the pulse cycle and width of the said first X-ray pulse and second X-ray pulse, so that the said first X-ray pulse and second X-ray pulse do not mutually interfere or have low interference degree in one cycle. Therefore, the synchronous real-time X-ray imaging of the said first imaging device and second imaging device can be realized. Further, the said imaging control device 7 can display the X-ray images in real time and synchronously by processing the said X-ray images.
In the embodiment, alternatively, the said first X-ray generator 3 is the first X-ray bulb tube and the said second X-ray generator 5 is the second X-ray bulb tube.
In the embodiment, alternatively, the said X-ray paradoxical pulse bi-planar synchronous real-time imaging device also includes the first motor 8 set on the said second C-arm 2. The said first motor 8 drives the said first C-arm to slide along the said second C-arm 2. The said first motor 8 is connected to the said console 71 by the signal of the motor controller 712 to control the motion of the said first motor 8 through the said console 71, hence to make different included angles of the said first imaging device and second imaging device.
In the embodiment, alternatively, the said X-ray paradoxical pulse bi-planar synchronous real-time imaging device also includes a foot switch 713, which is connected to the said console 71 by signal, so that the operating mode of the said X-ray paradoxical pulse bi-planar synchronous real-time imaging device can be selected through the foot switch 713. The said operating modes include: the first X-ray generator 3 and the first image receiver 4 operate; the second X-ray generator 5 and the second image receiver 6 operate; and the first X-ray generator 3 and the first image receiver 4 as well as the second X-ray generator 5 and the second image receiver 6 operate simultaneously.

Embodiment 2

By reference to Figure 1-4, this embodiment provides a method to realize the X-ray paradoxical pulse bi-planar synchronous real-time imaging by using the said X-ray paradoxical pulse bi-planar synchronous real-time imaging device, including the following steps:

S10. Control the motion of the said first motor 8 to make the included angle between the directions of the X-ray generated from the first X-ray generator 3 and the X-ray generated from the second X-ray generator 5 a preset value;
S20. Select the operating mode of the said X-ray paradoxical pulse bi-planar synchronous real-time imaging device;
S30. The said first high-voltage generator 74 and the second high-voltage generator 75 are controlled by the said X-ray controller 73 to generate the pulse high voltage. The said pulse high voltages generated by the said first high-voltage generator 74 and the second high-voltage generator 75 make the said first X-ray generator 3 and second X-ray generator 5 produce X-ray pulses, among which the X-ray pulse produced by the said first X-ray generator 3 is the first X-ray pulse and the X-ray pulse produced by the said second X-ray generator 5 is the second X-ray pulse.

The phase of the said first X-ray pulse and that of the second X-ray pulse are different;
S40. The said first image receiver 4 and second image receiver 6 respectively receive the X-ray pulses generated when the first X-ray pulse and second X-ray pulse penetrate the detection object and generate the visible light signals;
S50. The said first camera 77 and second camera 78 respectively collect the visible light signals generated by the first image receiver 4 and second image receiver 7 and transfer to the said image processor 72 by the network card 76;
S60. The said image processor 72 displays the visible light signals collected by the said first camera 77 on the first display 79 and visible light signals collected by the said second camera 78 on the second display 710.

The X-ray paradoxical pulse bi-planar synchronous real-time imaging method of the invention has the following beneficial effects: the said first X-ray generator 3 and second X-ray generator 5 are controlled by the said X-ray controller 73 to generate the first X-ray pulse and second X-ray pulse, among which the phases of the said first X-ray pulse and second X-ray pulse are different. Therefore, the said first X-ray pulse and second X-ray pulse do not mutually interfere or have low the interference degree in one cycle. Hence the synchronous real-time X-ray imaging of the said first imaging device and second imaging device can be realized. Further, the said imaging control device can display the X-ray images in real time and synchronously by processing the said X-ray images.

In the embodiment, alternatively, in S10, the said preset value is 5-95° to observe the detection objects such as human body and/or object from multi-angles.

In the embodiment, alternatively, in S20, the said operating modes include: a. The first X-ray generator 3 and the first image receiver 4 operate, while the second X-ray generator 5 and the second image receiver 6 do not operate; b. The second X-ray generator 5 and the second image receiver 6 operate, while the first X-ray generator 3 and the first image receiver 4 do not operate; c. The first X-ray generator 3 and the first image receiver 4 as well as the second X-ray generator 5 and the second image receiver 6 operate simultaneously, so that the said X-ray paradoxical pulse bi-planar synchronous real-time imaging method is suitable for different operating environments.

In the embodiment, alternatively, the said operation display is used to display the operation interface of the said X-ray paradoxical pulse bi-planar synchronous real-time imaging device and also supports the parameter input for the convenience of the user to operate the said X-ray paradoxical pulse bi-planar synchronous real-time imaging device.

In the embodiment, alternatively, there is also S05 before the S10:

S05. Set the cycle top limit T of the said X-ray paradoxical pulse bi-planar synchronous real-time imaging device to determine the low limit frequency of the said X-ray paradoxical pulse bi-planar synchronous real-time imaging device, so that to further improve the synchronism of the display of the said X-ray image. Preferably, the said T is not more than 1/24 second.

In the embodiment, alternatively, there is also S35 between the S30 and S40:

S35. The said first X-ray pulse's total cycle and the cycle top limit are the same, with the duration as $T_1$; the said second X-ray pulse's total cycle and the cycle top limit are the same, with the duration as $T_2$. The overlap between the said first X-ray pulse and the said second X-ray is Tt, satisfying $T_1 + T_2 \leq T + Tt$ to control the overlap amount of the pulse cycles of the said first X-ray pulse and second X-ray pulse. On the premise of increasing the energies of the said first X-ray pulse and second X-ray pulse, the interference between the said first X-ray pulse and second X-ray pulse reduces to further improve the imaging effect; preferably, the said $T_t$ meets:

$$T_t \leq 1/4 \times T_2.$$

Therefore, the interference between the said first X-ray pulse and second X-ray pulse can be further reduced.

In the embodiment, alternatively, there is also S36 between the S30 and S40: the said first X-ray pulse's total cycle and the cycle top limit are the same, with the duration as $T_1$; the said second X-ray pulse's total cycle and the cycle top limit are the same, with the duration as $T_2$, so that the said first X-ray pulse and second X-ray pulse do not mutually interfere. Preferably, the first X-ray pulse and second X-ray pulse are generated alternately.

In the embodiment, alternatively, in the said S35 or S36, the T1 and T2 are different, i.e., the energies of the said first X-ray pulse and second X-ray pulse are different. Then if the corresponding detection object of the first X-ray generator 3 is thicker, the $T_1$ can be prolonged properly and conversely, the $T_1$ can be reduced properly so that the examination effects of the thicker detection objects can be improved and the radiation dose of the thinner detection objects can be reduced.

In the embodiment, alternatively, the rising edge of the said second X-ray pulse is at or after the Tt prior to the falling edge of the said first X-ray pulse so that the overlap ratio of the said first X-ray pulse and second X-ray pulse can be controlled to improve the imaging quality. Preferably, the said $T_t \leq 1/4 \times T_2$ to avoid the said second X-ray pulse is covered by the said first X-ray pulse, resulting that the image after the detection object detected by the second X-ray generator 5 can't be output in high quality.

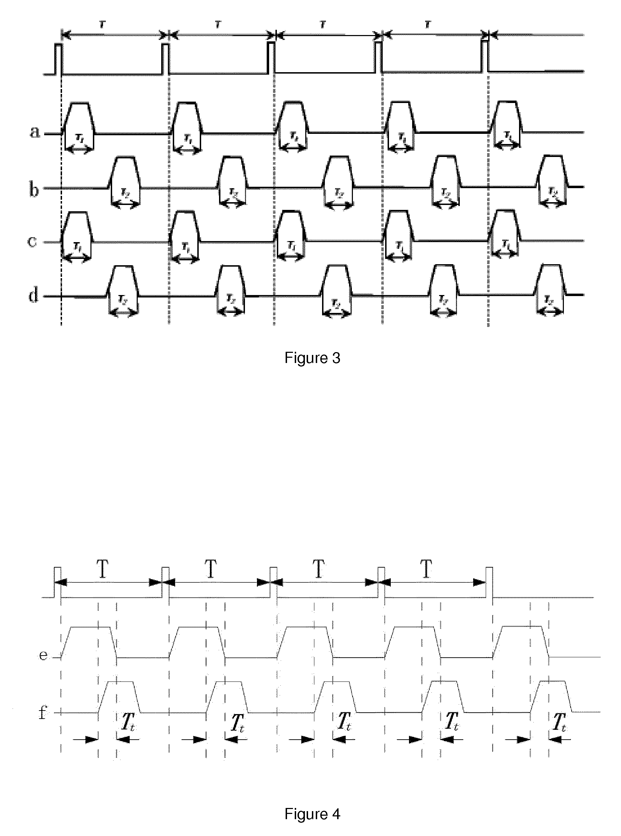

In the embodiment, alternatively, the time zero point of the said $T_1$ is at the midpoint of the rising edge of the said first X-ray pulse and the ending point is at the midpoint of the falling edge of the said first X-ray pulse; the time zero point of the said $T_2$ is at the midpoint of the rising edge of the said second X-ray pulse and the ending point is at the midpoint of the falling edge of the said second X-ray pulse; so that the time spans of the effective energy of the said first X-ray pulse and second X-ray pulse can be calculated accurately through the said $T_1$ and $T_2$, and hence the said X-ray paradoxical pulse bi-planar synchronous real-time imaging device can be controlled more precisely. In the embodiment, the Waveform a in attached Figure 3 is the sequence diagram of the first X-ray pulse; the Waveform b is the sequence diagram of the second X-ray pulse; the Waveform c is the sequence diagram of visible lights collected by the first camera; the Waveform d is the sequence diagram of visible lights collected by the second camera.

In the embodiment, the Waveform e in attached Figure 3 is the sequence diagram of the first X-ray pulse; the Waveform f is the sequence diagram of the second X-ray pulse.

The sequence of the above embodiments is only for ease of description, not representing the advantages and disadvantages of the embodiments.

Finally, it shall be noted that: the above embodiments are only used to explain the technical proposals of the invention, not limit the invention; despite the invention is described in detail by reference to the above embodiments, the ordinary technical personnel in this field shall understand that: they can still modify the technical proposals recorded in all embodiments above, or replace part of the technical features with the equivalent object; but these modifications or replacements shall not constitute the nature of the corresponding technical proposals out of the spirit and range of the technical proposals in the embodiments of the invention.

**Claims**

1. A kind of X-ray paradoxical pulse bi-planar synchronous real-time imaging device with the features including the first C-arm, the second C-arm, support device, the first X-ray generator, the first image receiver, the second X-ray generator, the second image receiver and imaging control device;
The said first C-arm is slidably set on the said second C-arm; the said second C-arm is slidably set on the said support device;
The said first X-ray generator and the first image receiver are fixed on two axial ends of the said first C-arm respectively;
The said second X-ray generator and the second image receiver are fixed on two axial ends of the said second C-arm respectively;
Taking the intersecting line between the x-ray emission plane of the said first X-ray generator and the x-ray emission plane of the said second X-ray generator as the rotation axis, the said first X-ray generator and the first image receiver rotate along the rotation axis driven by the said first C-arm and the said second X-ray generator and the second image receiver also rotate along the rotation axis driven by the said second C-arm;
The said imaging control device includes: console, image processor, X-ray controller, the first high-voltage generator, the second high-voltage generator, network card, the first camera, the second camera, the first display, the second display and operation display;
The said image processor and X-ray controller are connected to the signal of the said console; The said first high-voltage generator and second high-voltage generator are connected to the signal of the said X-ray controller and also connected to the first X-ray generator and the second X-ray generator respectively;
The said first camera and second camera are respectively connected to the said image processor and X-ray controller by the signal of the said network card;
The said first display, second display and operation display are connected to the signal of the said image processor respectively.

2. The X-ray paradoxical pulse bi-planar synchronous real-time imaging device, according to Claim 1, is **characterized in that** the said first X-ray generator is the first X-ray bulb tube and the said second X-ray generator is the second X-ray bulb tube.

3. The X-ray paradoxical pulse bi-planar synchronous real-time imaging device, according to Claim 2, is **characterized in that** it also includes the first motor set on the said second C-arm, which is connected to the said console by the motor control signal.

4. The X-ray paradoxical pulse bi-planar synchronous real-time imaging device, according to Claim 3, is **characterized in that** it also includes a foot switch, which is connected to the said console by signal.

5. The X-ray paradoxical pulse bi-planar synchronous real-time imaging device, according to Claim 4, is **characterized in that** it includes the following steps:

S10. Control the motion of the said first motor to make the included angle between the directions of the X-ray generated from the first X-ray generator and the X-ray generated from the second X-ray generator a preset value;
S20. Select the operating mode of the said X-ray paradoxical pulse bi-planar synchronous real-time imaging device;
S30. The said first high-voltage generator and the second high-voltage generator are controlled by the said X-ray controller to generate the pulse high voltage. The said pulse high voltages generated by the said first high-voltage generator and the second high-voltage generator make the said first X-ray generator and second X-ray generator produce X-ray pulses, among which the X-ray pulse produced by the said first X-ray generator is the first X-ray pulse and the X-ray pulse produced by the said second X-ray generator is the second X-ray pulse. The phase of the said first X-ray pulse and that of the second X-ray pulse are different;
S40. The said first image receiver and second image receiver respectively receive the X-ray pulses generated when the first X-ray pulse and second X-ray pulse penetrate the examined object and generate the visible light signals;
S50. The said first camera and second camera respectively collect the visible light signals generated by the first image receiver and second image receiver and transfer to the said image processor by the network card;
S60. The said image processor displays the visible light signals collected by the said first camera on the first display and visible light signals collected by the said second camera on the second display.

6. The X-ray paradoxical pulse bi-planar synchronous real-time imaging device, according to Claim 5, is **characterized in that** in the said S20, the said operating modes include:

a. The first X-ray generator and the first image receiver operate, while the second X-ray generator and the second image receiver do not operate;
b. The second X-ray generator and the second image receiver operate, while the first X-ray generator and the first image receiver do not operate;
c. The first X-ray generator and the first image receiver as well as the second X-ray generator and the second image receiver operate simultaneously.

7. The X-ray paradoxical pulse bi-planar synchronous real-time imaging device, according to Claim 5, is **characterized in that** there is also S05 before the S10:

S05. Set the cycle top limit T of the X-ray paradoxical pulse bi-planar synchronous real-time imaging device.

8. The X-ray paradoxical pulse bi-planar synchronous real-time imaging device, according to Claim 7, is **characterized in that** there is also S35 between the S30 and S40: the said first X-ray pulse's total cycle and the cycle top limit are the same, with the duration as $T_1$; the said second X-ray pulse's total cycle and the cycle top limit are the same, with the duration as $T_2$. The overlap between the said first X-ray pulse and the said second X-ray is Tt, satisfying $T_1 + T_2 \leq T + Tt$.

9. The X-ray paradoxical pulse bi-planar synchronous real-time imaging device, according to Claim 7, is **characterized in that** there is also S36 between the S30 and S40: the said first X-ray pulse's total cycle and the cycle top limit are the same, with the duration as $T_1$; the said second X-ray pulse's total cycle and the cycle top limit are the same, with the duration as $T_2$, satisfying $T_1 + T_2 \leq T$.

10. The X-ray paradoxical pulse bi-planar synchronous real-time imaging device, according to Claim 8 or 9, is **characterized in that** in the said S35 or S36, the $T_1$ and $T_2$ are different.

Figure 1

Figure 2

Figure 3

Figure 4

# INTERNATIONAL SEARCH REPORT

| International application No. |
| --- |
| PCT/CN2014/000057 |

### A. CLASSIFICATION OF SUBJECT MATTER

A61B 6/03 (2006.01) i; G01N 23/083 (2006.01) i; A61B 6/00 (2006.01) i; A61B 6/02 (2006.01) i

According to International Patent Classification (IPC) or to both national classification and IPC

### B. FIELDS SEARCHED

Minimum documentation searched (classification system followed by classification symbols)

A61B; G01N

Documentation searched other than minimum documentation to the extent that such documents are included in the fields searched

Electronic data base consulted during the international search (name of data base and, where practicable, search terms used)

CPRSABS, CNTXT, CNKI, VEN: x w ray, CT, biplane, biplanar, perpendicular+, transver+

### C. DOCUMENTS CONSIDERED TO BE RELEVANT

| Category* | Citation of document, with indication, where appropriate, of the relevant passages | Relevant to claim No. |
| --- | --- | --- |
| X | CN 1220134 A (OEC MEDICAL SYSTEMS INC.) 23 June 1999 (23.06.1999) the abstract, description, pages 7-12 and figure 2 | 1-10 |
| X | US 5515416 A (SICZEK et al.) 07 May 1996 (07.05.1996) description, column 2 and figures 1-6 | 1-10 |
| A | CN 203182923 U (GE MEDICAL SYSTEMS GLOBAL TECHNOLOGY CO., LTD.) 11 September 2013 (11.09.2013) the whole document | 1-10 |
| A | CN 102793552 A (BEIJING DONGFANG WHALE IMAGING TECHNOLOGY CO., LTD.) 28 November 2012 (28.11.2012) the whole document | 1-10 |
| A | CN 101194845 A (UNIV NANJING SCI & TECHNOLOGY et al.) 11 June 2008 (11.06.2008) the whole document | 1-10 |
| A | CN 103239250 A (THE FIRST AFFILIATED HOSPITAL OF THIRD MILITARY MEDICAL UNIVERSITY OF PLA) 14 August 2013 (14.08.2013) the whole document | 1-10 |

☒ Further documents are listed in the continuation of Box C.     ☒ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&"document member of the same patent family |

| Date of the actual completion of the international search | Date of mailing of the international search report |
| --- | --- |
| 16 October 2014 | 02 November 2014 |

| Name and mailing address of the ISA State Intellectual Property Office of the P. R. China No. 6, Xitucheng Road, Jimenqiao Haidian District, Beijing 100088, China Facsimile No. (86-10) 62019451 | Authorized officer ZHANG, Qingnan Telephone No. (86-10) 62085610 |
| --- | --- |

Form PCT/ISA /210 (second sheet) (July 2009)

**INTERNATIONAL SEARCH REPORT**

| International application No. |
| --- |
| PCT/CN2014/000057 |

C (Continuation).     DOCUMENTS CONSIDERED TO BE RELEVANT

| Category* | Citation of document, with indication, where appropriate, of the relevant passages | Relevant to claim No. |
| --- | --- | --- |
| A | CN 201572102 U (BEIJING DONGFANG WHALE IMAGING TECHNOLOGY CO., LTD.) 08 September 2010 (08.09.2010) the whole document | 1-10 |
| A | US 4426725 A (GRADY JOHN K) 17 January 1984 (17.01.1984) the whole document | 1-10 |

Form PCT/ISA /210 (continuation of second sheet) (July 2009)

## INTERNATIONAL SEARCH REPORT
### Information on patent family members

| International application No. |
| --- |
| PCT/CN2014/000057 |

| Patent Documents referred in the Report | Publication Date | Patent Family | Publication Date |
| --- | --- | --- | --- |
| CN 1220134 A | 23 June 1999 | AT 265181 T | 15 May 2004 |
| | | US 6104780 A | 15 August 2000 |
| | | JP 2000157527 A | 13 June 2000 |
| | | EP 0917856 B1 | 28 April 2004 |
| | | DE 69823456 T2 | 19 May 2005 |
| | | JP 4424560 B2 | 03 March 2010 |
| | | DE 69823456 D1 | 03 June 2004 |
| | | CN 1174714 C | 10 November 2004 |
| | | EP 0917856 A1 | 26 May 1999 |
| US 5515416 A | 07 May 1996 | None | |
| CN 203182923 U | 11 September 2013 | None | |
| CN 102793552 A | 28 November 2012 | US 2014086384 A1 | 27 March 2014 |
| | | WO 2012159279 A1 | 29 November 2012 |
| | | CN 102793552 B | 21 May 2014 |
| CN 101194845 A | 11 June 2008 | None | |
| CN 103239250 A | 14 August 2013 | None | |
| CN 201572102 U | 08 September 2010 | None | |
| US 4426725 A | 17 January 1984 | GB 2120060 A | 23 November 1983 |
| | | GB 2120060 B | 11 September 1985 |
| | | DE 3316719 A1 | 17 November 1983 |
| | | FR 2526300 A2 | 10 November 1983 |

Form PCT/ISA /210 (patent family annex) (July 2009)

**EP 3 047 799 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4884293 A
- EP 0917856 A1